# EUROPEAN PATENT APPLICATION

(11) **EP 4 541 412 A1**
(43) Date of publication of application: **23.04.2025**
(21) Application number: 23204218.4
(22) Date of filing: 17.10.2023
(51) Int. Cl.: A61N 1/05

(54) **DEVICE FOR ELECTRICAL NERVE STIMULATION AND/ OR MAPPING OF BODY TISSUE**

(71) Applicant: Cryovasc GmbH, 10119 Berlin (DE)
(72) Inventor: HEINER, Wilfred Peter, 10119 Berlin (DE)
(74) Representative: Stütz, Jan

(57) **Abstract**

Device for electrical nerve stimulation and/or mapping, of body tissue comprising a guide wire with a proximal and a distal end and at least one electrode or sensor on the distal end, characterized in that the guide wire comprises at least one spiral /helical shaped groove along the guide wire.

## Description

Ablation procedures are used to modify the electrophysical properties of nerve cells by inactivating them by means of laser, radiofrequency therapy or cryoablation. As a result, they can neither generate nor transmit electrical impulses. This procedure is used, for example, to treat cardiac arrhythmias or hypertension. Special nerve cells in the heart or kidneys are ablated for this purpose.

To improve the efficiency of ablation processes it might be advantageously to check whether the vessels intended for ablation still can transmit stimuli or whether the treatment was successful in the vessels that have already been ablated. This enables, for example, to test the reaction of the nerves by applying a stimulus.

Document US20140276124A1 describes for example methods and devices of determining efficacy of a renal denervation procedure in a renal artery. The method includes providing a renal denervation catheter having at least one ablation member and at least one vibration sensor, operating the at least one ablation member to provide renal denervation in the renal artery, measuring vibrations in the renal denervation catheter prior to and after providing the renal denervation, and comparing the measured vibrations to determine a change in blood flow rate through the renal artery as an indicator of efficacy of the renal denervation. The vibration sensor may be carried by a wire member insertable through a lumen of the catheter shaft. The vibration sensor may be positioned at the distal end of a deployable basket carrying the electrodes. A controller may be directly wired to the ablation electrodes or may have wireless communication.

US20140276124A1 relates to an ablation device comprising a temperature monitoring system. The temperature monitoring system may include at least one temperature sensor and is configured to determine a change in temperature in a blood flow through the renal artery. The change in temperature correlates to a rate of blood flow.

Document WO2017223264A1 and document US2017252101A1 disclose a method of evaluating the intraprocedural success of a renal denervation procedure on a subject. The method comprises: electrically stimulating multiple points about the circumference of a renal artery of a subject using at least one spline of an electrode assembly as a stimulation electrode to determine a baseline physiological response of the subject before and after renal denervation. The documents further describe an electrode assembly that includes multiple splines that extend from an insulated proximal hub to an insulated distal hub and are interconnected to an electrical wire to allow the splines to independently function as electrical stimulation electrodes.

All these documents describe devices for measuring denervation wherein the electrodes being positioned in a basket.

In document US2013274614A1 a method for positioning the stimulation electrode against the wall of the artery at a first or a second location, measuring blood velocity, then emitting an electrical pulse, then measuring blood velocity again at a first or a second electrode is described.

The physiological parameter, or the change in the physiological parameter due to stimulation, may be measured before, during and after ablation. The measurement of the physiological parameter, or the change in the physiological parameter due to stimulation is a way of monitoring the effectiveness of the ablation.

Beside other electrode configurations it is shown that numerous electrodes are located on a spiral shaped distal end of the catheter. The catheter distal end spiral may be sized to about the inner surface of a renal artery throughout its loops to allow for mapping around the artery without, or with less, repositioning of the catheter.

Document CN114305654A describes an ablation device for sympathetic nerve ablation. The head of the ablation device being provided with control arms comprising two detection electrodes, and an ablation electrode between the two detection electrodes and an insulation between the adjacent detection electrodes and the ablation electrodes. An ablation body is connected between the handle and the head of the connection device. The connecting cables are arranged in the ablation tube body comprising a sheath.

The devices of the state in the art are not completely satisfactory. They are sometimes bulky, not easy to handle.

The task of the invention is providing a device for electrical nerve stimulation and mapping e.g., for measuring denervation which overcomes the disadvantageous of the devices of the state in the art. This task is solved by a device according to claim 1, a method of claim 9 and a system of claim 10.

The field of application of the invention is medicine. The invention relates to a guide wire to guide catheters and other medical devices into blood vessels or other hollow structures of the body, characterized in that the guide wire is grooved in a spiral/ helical shape along the guide wire.

Preferably the guide wire comprises at least one electrical wire embedded in the at least one spiral/ helical groove. Preferably at least one electrical wire and/or the guidewire is coated with a sleeve. Most preferred this sleeve is a polymer sleeve.

In a special embodiment of the invention the guide wire is part of a medical system. In such a system a medical console/ controller or other electrical device might be connected to the proximal end of the guide wire via at least one electrical wire.

According to another aspect, the guide wire forms part of an ablation device. According to a preferred embodiment, the ablation device is a cryoablation device.

On the distal end of the guide wire at least one electrode and or sensor is located. It is preferably connected to the electrical wire or wires. One or more electrical wires can be arranged in the spiral /helical shaped groove. One or more grooves can be arranged preferably in parallel along the guide wire.

Preferably the proximal end of the guide wire comprises a connection for connecting the guidewire to an electrical source. The at least one electrical wire connects the medical device to the electrical source.

Especially, the invention relates to a device for measuring denervation comprising a guide wire with a proximal and a distal end and at least one preferably at least two stimulation electrodes, characterized in that the guide wire is grooved in a spiral/ helical shape along the guide wire and comprises at least one pre-shaped loop at the distal end.

In a preferred embodiment of the invention the device further comprises at least one sensor; this might be a pressure sensor.

The device according to the invention preferably comprises at least one stimulation electrode. The at least one stimulation electrode might be placed in the at least one pre- shaped loop.

In a preferred embodiment of the invention the device comprises at least two stimulation electrodes which are preferably placed at a distance from each other on that at least one pre-shaped loop. If the device according to the invention comprises more than two stimulation electrodes, the electrodes might be positioned circumferentially and preferably uniformly distributed at different locations at the at least one pre-shaped loop.

Advantageously, the guide wire consists of a flexible material, especially preferred is Nitinol. Preferably, the device according to the invention comprises at least one electrode on the proximal side of the guide wire which can be connected to a pulse generator. This electrode is intended to send a stimulation pulse to the at least one stimulation electrode located on the pre- shaped loop.

Preferably, the device according to the invention further comprises at least one electrical wire or cable to transmit the electrical signal to and from the electrodes and, if applicable, from the blood pressure measurement sensor or sensing electrode. According to the invention, the at least one electrical wire is arranged in the spiral/ helical shaped groove or grooves of the guide wire.

In a preferred embodiment, the cables connect the electrodes and, if necessary, the sensors to a control unit, which controls the stimulation by and the power of the electrodes as well as the sensor element, if present.

In another preferred embodiment of the invention, the guide wire and the at least one electrical wire is coated.

In a preferred embodiment of the invention, the guide wire is coated with a preferably insulating material, preferably a polymer sleeve, which is keeping the wires in place and/ insulates the wire(s). Most preferably the polymer sleeve comprises or consists of ethylene tetrafluoroethylene. In a special embodiment of the invention also a heat shrinkable sleeve might be used. Any interruptions in the isolation with the heat shrinkable sleeve can preferably be filled with adhesive, which then takes over the isolation at this point. This coat is the isolation between the electrodes and the guidewire and between the electrodes and the electrodes and the grooved wire. This construction is stable, simple, space-saving and easy to handle.

The device of present invention is adaptable for use in different vessels. Preferably, the diameter of the at least one pre-shaped loop and the location of the electrodes is adapted to the diameter of the vessel for measuring the denervation. In other words, the diameter of the at least one pre-shaped loop and the distribution of the electrodes correspond to the diameter of the vessel. For renal denervation this will be a diameter between 3 and 8 mm. Other diameters, for example for the use in other vessels, are also possible and the corresponding construction of the device according to the invention is easy to implement.

Several sizes for the guide wire can be applied. The guide wire of the device is preferably a 014-guide wire but can be constructed as a 025, 035 or 038 guide wire, too, as an example.

In a special embodiment of the invention, the at least one stimulation electrode is a unipolar stimulation electrode or is used as a unipolar stimulation electrode. In this case, the device of the present invention preferably further comprises at least one electrode connected to an impulse generator which is preferably located proximal from the stimulation electrode.

In this case the at least one stimulation electrode is connected to the at least one electrode connected to an impulse generator via at least one electrical conducting wire located in the helical groove of the guide wire.

In a preferred embodiment, more than one unipolar or bipolar stimulation electrodes and more than one impulse generator connected electrodes are used to achieve a reliable stimulation of the nerve, which in most of the cases surrounds the blood vessel.

In another preferred embodiment of the invention, the at least one stimulation electrode is at least two bipolar stimulation electrodes or is at least one electrode pair, which is used as unipolar polar stimulation electrodes, respectively.

In another preferred embodiment of the invention, the device might further comprise bipolar and unipolar stimulation electrodes.

The invention further relates to a method of electrical nerve stimulation comprising the following steps:
- contacting the at least one stimulation electrode of the guide wire described above to the wall of a vessel;
- electrically stimulating the nerve surrounding the vessel.

To determine denervation in a tissue the method might further comprise measuring the blood pressure of a patient before contacting the at least one stimulation electrode of the guide wire to the wall of a vessel and electrically stimulating the nerve surrounding the vessel as well as measuring of the blood pressure of this patient after this step.

Thereby the sensor for measuring the blood pressure of the patient might be part of the device according to the invention. In another preferred embodiment the sensor is not part of the device according to the invention and the measurement is carried out independently from the device.

If the measurement shows no or minimal change in blood pressure of the patient, it can be assumed that the ablation has been effective, and the nerves are inactivated.

The method can be performed prior to or after ablation. If the method is performed before ablation, it can be checked, if ablation is necessary at the tested location. By performing the method after the ablation process, it can be figured out, if the ablation was successful or if it has to be repeated at the respective location.

The invention thus also relates to a use for the device according to the invention for measuring denervation of nerve cells of a human or an animal body.

One big advantage of the device according to the invention is that it can be used independently from the ablation device. It can be used before and/ or after the ablation device is used. It can be inserted into the vessel of interest independently from the ablation device. It can be used also together with the most various types of ablation devices and be combined with them, if applicable.

Therefore, the invention further relates to a system comprising the device according to the invention and a device for ablation nerve cells.

In some embodiments of the invention, it is possible that the guide wire with the at least one stimulation electrode is arranged along the catheter containing the ablation electrodes in case of radio frequency ablation or the cryoablation catheter, for example.

Preferably the pre-shaped loop forms the tip part of such a device and the ablation system is located proximal to it on the catheter shaft.

In another special embodiment of the invention, the ablation electrodes of a radio frequency ablation device, for example, or another ablation device and the at least one stimulation electrode of the device according to the invention can be combined in that way, that both are located on the guide wire. In this case the ablation electrodes could be electrically connected via the guide wire body.

Further details can be obtained from the description of the figures, which represent preferred embodiments of the invention showing:
Fig. 1 cross sectional view of the guide wire;
Fig. 2 isometric view of the guide wire with electrodes for unipolar stimulation;
Fig. 3 isometric view of the guide wire with electrodes for bipolar stimulation.

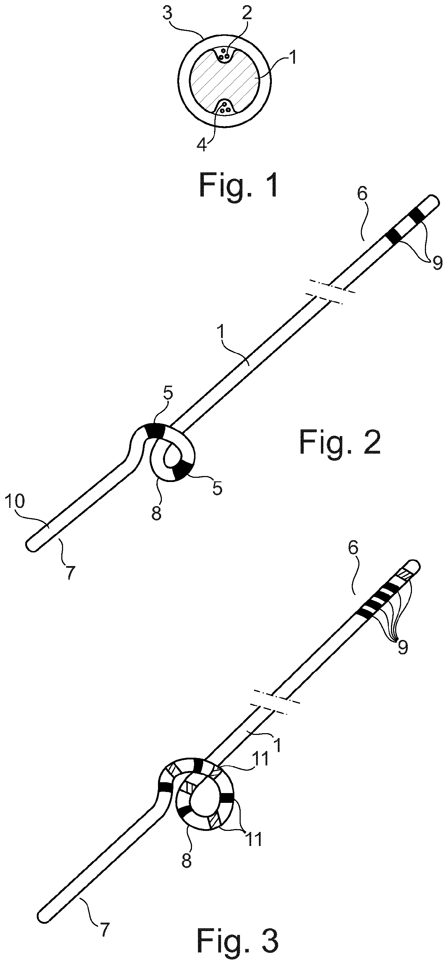

Figure 1 shows a cross sectional view of the guide wire 1 comprising a spiral /helical groove 2 and a coat 3 as well as electrical wires or cables 4 that are proximal and distal connected to electrodes.

The electrical wires 4 are embedded in the spiral groove 2 of the guide wire 1 and coated by a polymer sleeve. Thus, the electrical wires 4 are compactly fixed and a slipping of the electrical wires 4 is impossible. This design enables to reduce the combined diameter of guide wire and wires. The polymer coat 3 represents the outer sheath and at the same time the isolation between the electrodes and the electrodes and the guide wire 4.

Figure 2 shows an isometric view of the guide wire 1 with electrodes for unipolar stimulation 5. The proximal end 6 and the distal end 7 of the guide wire 1 are shown. The distal end of the guide wire 1 comprise a pre-shaped loop 8 where two stimulation electrodes for unipolar stimulation 5 are placed.

At the proximal end 6 of the guide wire 1 the impulse generator connected electrodes 9 are positioned. the proximal electrodes connected to the pulse generator 9 apply a stimulus and transfer it to the unipolar stimulation electrodes 5. The stimulation electrodes 5 stimulate the nerve surrounding the vessel two different locations. If the ablation was successful, no or only minimal change in blood pressure occurs, which can be measured with a sensor [not shown).

Figure 3 shows an isometric view of the guide wire 1 with electrodes for bipolar stimulation 11. The bipolar stimulating electrodes 11 are placed alternately on the pre-shaped loop 8 of the guide wire 1. More than one bipolar stimulating electrode pair is possible.

At the proximal end 6, electrodes connected to the pulse generator 9 are arranged. The bipolar stimulating electrodes are connected to the pulse generator connected electrodes 9 via the electrical wires 4 embedded in the spiral/ helical groove 2 of the guide wire 1 The stimulation electrodes 11 of the pre-shaped loop 8 are corresponding electrode pairs. The bipolar stimulating electrodes 11 of the pre-shaped loop 8 consists of at least one electrode pair comprising cathode or base electrode and anode. A base electrode 9 is located at the proximal end 6 of the guide wire. In case of activation of the electrodes, current flows between the cathode or base electrode and the anode of and a change or no change, respectively, in the blood pressure can be measured, depending on the condition of the nerve cells effected by electrical stimulation caused by the electrodes.

## Claims

1. Device for electrical nerve stimulation and/or mapping, of body tissue comprising a guide wire with a proximal and a distal end and at least one electrode or sensor on the distal end, **characterized in that** the guide wire comprises at least one spiral /helical shaped groove along the guide wire.

2. Device of claim 1, **characterized in that** the at least one electrode is placed on at least one pre- shaped loop at the distal end of the guide wire.

3. Device of any of the foregoing claims further comprising at least one electrical wire, **characterized in that** the at least one electrical wire is arranged in the at least one spiral/ helical shaped groove of the guide wire.

4. Device of any of claim 3, **characterized in that** the guide wire and the at least one electrical wire is coated.

5. Device of any of the claims 2-4, **characterized in that** the diameter of the at least one pre-shaped loop and the location of the at least one stimulation electrode is adapted to the diameter of the vessel.

6. Device of any of the foregoing claims, **characterized in that** the at least one stimulation electrode is used as a unipolar stimulation electrode.

7. Device of any of the foregoing claims, **characterized in that** the at least two electrodes are used as bipolar stimulation electrodes.

8. Device of any of the foregoing claims, **characterized in that** the device further comprises at least one sensor.

9. Method of electrical nerve stimulation comprising the following steps
• contacting the at least one stimulation electrode of the above-described guide wire to the wall of a vessel;
• electrically stimulating the nerve surrounding the vessel.

10. System comprising the device of claim 1-8 and a device for ablation of nerve cells.
